# EUROPEAN PATENT APPLICATION

(11) **EP 0 695 802 A2**
(43) Date of publication of application: **07.02.1996**
(21) Application number: 95303678.7
(22) Date of filing: 30.05.1995
(51) Int. Cl.: C12N 15/12, C07K 14/72, C07K 16/26, G01N 33/68, C12N 15/00, A01K 67/027, C12Q 1/68

(54) **Human CRH receptor-related receptor**

(30) Priority: 10.06.1994 US 259265
(71) Applicant: PFIZER INC., New York, N.Y. 10017 (US)
(72) Inventor: De Wet, Jeffrey R., Pawcatuck, Connecticut 06379 (US)
(74) Representative: Moore, James William, Dr.

(57) **Abstract**

This invention relates to a novel nucleic acid sequence that encodes a portion of a novel human CRH receptor-related G protein-coupled receptor, and to the amino acid sequence that it encodes.

SEQUENCE LISTING

(1) GENERAL INFORMATION:
(i) APPLICANT: PFIZER INC
(ii) TITLE OF INVENTION: HUMAN CRH RECEPTOR RELATED RECEPTOR
(iii) NUMBER OF SEQUENCES: 4
(iv) CORRESPONDENCE ADDRESS:
(A) ADDRESSEE: PFIZER INC.
(B) STREET: 235 EAST 42ND STREET, 20TH FLOOR
(C) CITY: NEW YORK
(D) STATE: NEW YORK
(E) COUNTRY: U.S.A.
(F) ZIP: 10017-5755
(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25
(vi) CURRENT APPLICATION DATA:
(A) APPLICATION NUMBER: US N/A
(B) FILING DATE:
(C) CLASSIFICATION:
(viii) ATTORNEY/AGENT INFORMATION:
(A) NAME: DEBENEDICTIS, KAREN
(B) REGISTRATION NUMBER: 32,977
(C) REFERENCE/DOCKET NUMBER: PC8734
(ix) TELECOMMUNICATION INFORMATION:
(A) TELEPHONE: (212)573-5425
(B) TELEFAX: (212)573-1939
(C) TELEX: N/A
(2) INFORMATION FOR SEQ ID NO:1:
(i) SEQUENCE CHARACTERISTICS:
(A) LENGTH: 471 base pairs
(B) TYPE: nucleic acid
(C) STRANDEDNESS: double
(D) TOPOLOGY: linear
(ii) MOLECULE TYPE: cDNA
(xi) SEQUENCE DESCRIPTION: SEQ ID NO:1:
ATGTTTGTGG AAGGCTGCTA CCTGCACACG GCCATTGTCA TGACCTACTC CACTGAGCGC 60
CTGCGCAAGT GCCTCTTCCT CTTCATCGGA TGGTGCATCC CCTTCCCCAT CATCGTCGCC 120
TGGGCCATCG GCAAGCTCTA CTATGAGAAT GAACAGTGCT GGTTTGGCAA GGAGCCTGGC 180
GACCTGGTGG ACTACATCTA CCAAGGCCCC ATCATTCTCG TGCTCCTGAT CAATTTCGTA 240
TTTCTGTTCA ACATCGTCAG GATCCTAATG ACAAAGTTAC GCGCGTCCAC CACATCCGAG 300

ACAATCCAGT ACAGGAAGGC AGTGAAGGCC ACCCTGGTGC TCCTGCCCCT CCTGGGCATC 360
ACCTACATGC TCTTCTTCGT CAATCCCGGG GAGGACGACC TGTCACAGAT CATGTTCATC 420
TATTTCAACT CCTTCCTGCA GTCGTTCCAG GGTTTCTTCG TGTCTGTCTT C 471
(2) INFORMATION FOR SEQ ID NO:2:
(i) SEQUENCE CHARACTERISTICS:
(A) LENGTH: 157 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:2:
Met Phe Val Glu Gly Cys Tyr Leu His Thr Ala Ile Val Met Thr Tyr
1               5                   10                  15
Ser Thr Glu Arg Leu Arg Lys Cys Leu Phe Leu Phe Ile Gly Trp Cys
            20                  25                  30
Ile Pro Phe Pro Ile Ile Val Ala Trp Ala Ile Gly Lys Leu Tyr Tyr
        35                  40                  45
Glu Asn Glu Gln Cys Trp Phe Gly Lys Glu Pro Gly Asp Leu Val Asp
    50                  55                  60
Tyr Ile Tyr Gln Gly Pro Ile Ile Leu Val Leu Leu Ile Asn Phe Val
65                  70                  75                  80
Phe Leu Phe Asn Ile Val Arg Ile Leu Met Thr Lys Leu Arg Ala Ser
                85                  90                  95
Thr Thr Ser Glu Thr Ile Gln Tyr Arg Lys Ala Val Lys Ala Thr Leu
            100                 105                 110
Val Leu Leu Pro Leu Leu Gly Ile Thr Tyr Met Leu Phe Phe Val Asn
        115                 120                 125
Pro Gly Glu Asp Asp Leu Ser Gln Ile Met Phe Ile Tyr Phe Asn Ser
    130                 135                 140
Phe Leu Gln Ser Phe Gln Gly Phe Phe Val Ser Val Phe
145                 150                 155
(2) INFORMATION FOR SEQ ID NO:3:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: cDNA
(xi) SEQUENCE DESCRIPTION: SEQ ID NO:3:
GTTTGGCAAG GAGCCTGGCG AC
22
(2) INFORMATION FOR SEQ ID NO:4:
(i) SEQUENCE CHARACTERISTICS:

(A) LENGTH: 19 base pairs
(B) TYPE: nucleic acid
(C) STRANDEDNESS: single
(D) TOPOLOGY: linear
(ii) MOLECULE TYPE: cDNA
(xi) SEQUENCE DESCRIPTION: SEQ ID NO:4:
CATGATCTGT GACAGGTCG
19

## Description

This invention relates to a nucleic acid sequence that encodes a portion of a novel human corticotropin releasing hormone (CRH) receptor-related G protein-coupled receptor and to the peptide sequence that it encodes. Such peptide sequence is highly homologous to the cloned human CRH receptor, as described by Chen et al., Proc. Nat'l Acad. Sci. U.S.A., 90, 8967-71 (1993) and Chang et al., Neuron, 11, 1189-95 (1993), but shows significant changes in its predicted amino acid sequence. The novel nucleic acid sequence of this invention and subsets of it may be used as probes or PCR primers to isolate the full length cDNA encoding a human CRH receptor- related G protein-coupled receptor, which can then be expressed in eucaryotic or prokaryotic cells for the purpose of discovering antagonists or agonists useful in developing therapeutic compounds. The high degree of homology between the CRH receptor-related receptor of this invention (a putative CRH receptor subtype) and the previously described human CRH receptor indicates that such a clone will be useful in developing CRH receptor ligands with greater specificity of action and fewer undesirable side effects compared to known CRH receptor ligands.

Corticotropin releasing hormone (CRH) is a 41 amino acid peptide that was originally isolated from ovine hypothalamus extracts. It has been referred to in the literature, prior to its characterization as a hormone, as corticotropin releasing factor (CRF). As reported by Vita et al., Federation of European Biochemical Societies, 335 (1), 1-5 (1993), CRH-producing neurons, which have their origin in the parvocellular region of the paraventricular nucleus of the hypothalamus, are the major physiological regulators of the basal and stress-induced secretion of adrenocorticotropin hormone (ACTH), β-endorphin and other proopiomelanocortin-related peptides from the anterior pituitary.

Chen et al., Proc. Nat. Acad. Sci., 90, 8967-8971 (1993), report that anatomic, functional, and pharmacologic studies have suggested numerous physiologic roles for CRH within the brain, adrenals, gonads, gastrointestinal tract, placenta, and sites of inflammation, and that a subset of CRH neurons may coordinate many of the endocrine, autonomic, and behavioral responses to stress and may be involved in the pathophysiology of affective disorders. They also report that CRH appears to be a key intermediary in the communication between the immune systems and the central nervous and endocrine systems. CRH and its binding sites are broadly distributed throughout the central nervous system as well as in some peripheral sites, reflecting the role CRH may play in endocrine, autonomic, and behavioral aspects of the stress response, as well as in such pathophysiologic states as depression, anxiety, and anorexia nervosa. (See Chen et al., Proc. Natl. Acad Sci., 90, 8967-71 (1993)).

CRF exerts its effects by initially binding to a membrane bound receptor. High-affinity CRF binding sites have been characterized in the rat and human pituitary, rat and human brain, and in AtT20 cells, a mouse corticotropic tumor cell line. (See Chen et al., Proc. Natl. Acad Sci., 90, 8967-71 (1993)). The CRH receptor is coupled to adenylate cyclase, probably through a GTP-binding protein. (See Chen et al., Proc. Natl. Acad Sci., 90, 8967-71 (1993)).

The above articles by Chen et al., and Vita et al., are incorporated herein by reference in their entireties.

Chen et al., in the reference discussed above, report the cloning of a cDNA that encodes for a 415 amino acid CRF receptor from a human corticotropic tumor library.

CRF ligands that are peptides and pyrazolinones are mentioned, respectively, in U.S. Patents 4,605,642 and 5,063,245 referring to peptides and pyrazolinones, respectively.

The importance of CRF ligands is discussed in the literature, e.g., as discussed in U.S. Patent 5,063,245, which is also incorporated herein by reference in its entirety. A recent outline of the different activities possessed by CRF antagonists appears in an article by Owens et al., Pharm. Rev., 43, 425-473 (1991). This article is also incorporated herein by reference in its entirety. Based on the research described in these two and other references, CRF antagonists are considered effective in the treatment of a wide range of diseases including stress-related illnesses such as stress-induced depression, anxiety, and headache, anorexia nervosa, irritable bowel syndrome, spastic colon, inflammatory diseases, immune suppression, human immunodeficiency virus (HIV) infections, Alzheimer's disease, gastrointestinal diseases, anorexia nervosa, hemorrhagic stress, drug and alcohol withdrawal symptoms, drug addiction, and fertility problems.

This invention relates to a nucleic acid sequence encoding a 157 amino acid portion of a novel human corticotropin releasing hormone (CRH) receptor related G protein-coupled receptor. More specifically, it relates to an isolated DNA segment comprising the DNA sequence of SEQUENCE ID NO. 1, as defined below, or an allelic variation of such sequence.

This invention also relates to a substantially purified polypeptide produced by expression in a host cell into which has been incorporated the DNA sequence of SEQUENCE ID NO. 1 or an allelic variation of such sequence.

This invention also relates to a substantially purified polypeptide comprising the amino acid sequence of SEQUENCE ID NO. 2. It also relates to a substantially purified polypeptide that is functionally equivalent to a polypeptide comprising the amino acid sequence of SEQUENCE ID NO. 2.

This invention also relates to recombinant DNA comprising the DNA sequence of SEQUENCE ID NO. 1 or an allelic variation of such sequence.

This invention also relates to a DNA vector comprising recombinant DNA comprising the DNA sequence of SEQUENCE ID NO. 1 or an allelic variation of such sequence. It also relates to a host cell into which has been incorporated recombinant DNA comprising the DNA sequence of SEQUENCE ID NO. 1 or an allelic variation of such sequence.

This invention also relates to an isolated DNA segment comprising a DNA sequence that is a subset of the DNA sequence of SEQUENCE ID NO. 1, or an allelic variation of such sequence, and that is capable of hybridizing to the DNA sequence of SEQUENCE ID NO. 1, or an allelic variation of such sequence, when used as a probe, or of amplifying all or part of such sequence when used as a polymerase chain reaction primer.

The invention also relates to a method of detecting the presence in human cells of mRNA that encodes human CRH receptor-related receptor that comprises the peptide sequence of SEQUENCE ID NO. 2, comprising: (a) performing a reverse transcriptase-polymerase chain reaction on total RNA from human cells using a pair of polymerase chain reaction primers that are specific for such receptor, as determined from the DNA sequence of SEQUENCE ID NO. 1 or an allelic variation of such sequence, and (b) assaying the presence of an appropriately sized PCR fragment, or hybridizing the products of such reaction to the DNA sequence of SEQUENCE ID NO. 1.

This invention also relates to a method of identifying ligands that bind to or displace the binding of a known ligand to a human CRH receptor-related receptor that comprises the peptide sequence of SEQUENCE ID NO. 2, comprising determining the degree of binding of a substance to such receptor or determining the ability of a substance to displace the binding of a known ligand (e.g., CRH) to such receptor in (a) a cell line into which has been incorporated recombinant DNA comprising the DNA sequence of SEQUENCE ID NO. 1 or an allelic variation of such sequence, or (b) a cell population or cell line that naturally selectively expresses such receptor.

This invention also relates to a method of isolating a full length human CRH receptor-related receptor cDNA or a human genomic CRH receptor-related receptor clone, comprising using the DNA sequence of SEQUENCE ID NO. 1 or an allelic variation thereof, or a subset of such sequence or allelic variation of such sequence, as a probe to screen a human cDNA or genomic library, or as a polymerase chain reaction primer.

This invention also relates to a method of modulating expression in a human cell of a human CRH receptor-related receptor gene that comprises the DNA sequence of SEQUENCE ID NO. 1 or an allelic variation of such sequence, comprising synthesizing an antisense oligonucleotide complimentary to the coding region of human CRH receptor-related receptor mRNA, and treating a human cell line in culture or in vivo with such oligonucleotide.

As used herein, the term "functionally equivalent DNA segment" refers to a DNA segment that encodes a polypeptide having an activity that is substantially the same as the activity of the polypeptide encoded by the DNA to which such segment is said to be functionally equivalent.

As used herein, the term "subset of a DNA sequence" refers to a nucleotide sequence that is contained in and represents part, but not all of such DNA sequence, and is sufficient to render it specific to such sequence when used as a PCR primer and render it capable of hybridizing to such sequence when used as a probe at high stringency.

As used herein, the term "functionally equivalent polypeptide" refers to a polypeptide that has substantially the same activity as the polypeptide to which it is said to be functionally equivalent.

The term "functionally equivalent peptide", as used herein, refers to a peptide sequence that shares with the peptide sequence of SEQUENCE ID NO. 2 the ability to produce a monoclonal or polyclonal antibody against a CRH receptor-related receptor that comprises the peptide sequence of SEQUENCE ID NO. 2 in an animal that is immunized with the peptide that is said to be a functional equivalent.

The term "subset of a peptide sequence" refers to a smaller peptide sequence that is contained in and represents part, but not all of the peptide sequence that appears in such term.

### Brief Description of the Drawings

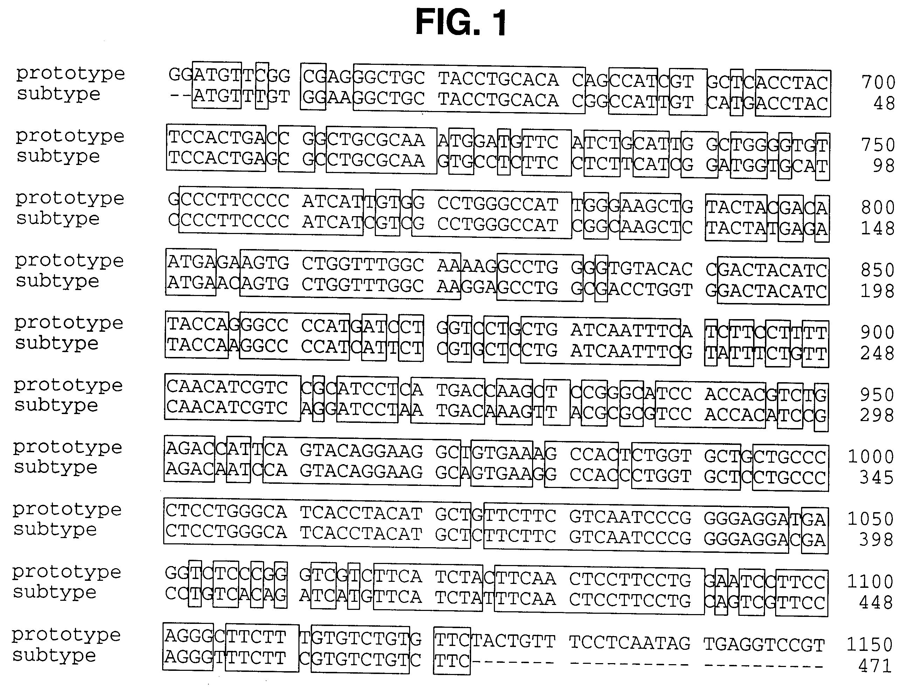

Fig. 1. Sequence alignment of the CRH receptor related cDNA with the prototype human CRH receptor. The sequence that was determined for the CRH receptor related cDNA clones (labeled subtype) was aligned with the sequence of the human CRH receptor (Chen et al., 1993) labeled "prototype" using the program GeneWorks (Intelligenetics, Inc., Mountain View, California). The numbering for the prototype receptor is according to the sequence entry in Genbank (National Institute of Health, Maryland), locus name HUMCRFRA, accession number L23332. Identities between the two sequences are boxed, and within the regions compare, these sequences are 82% homologous.

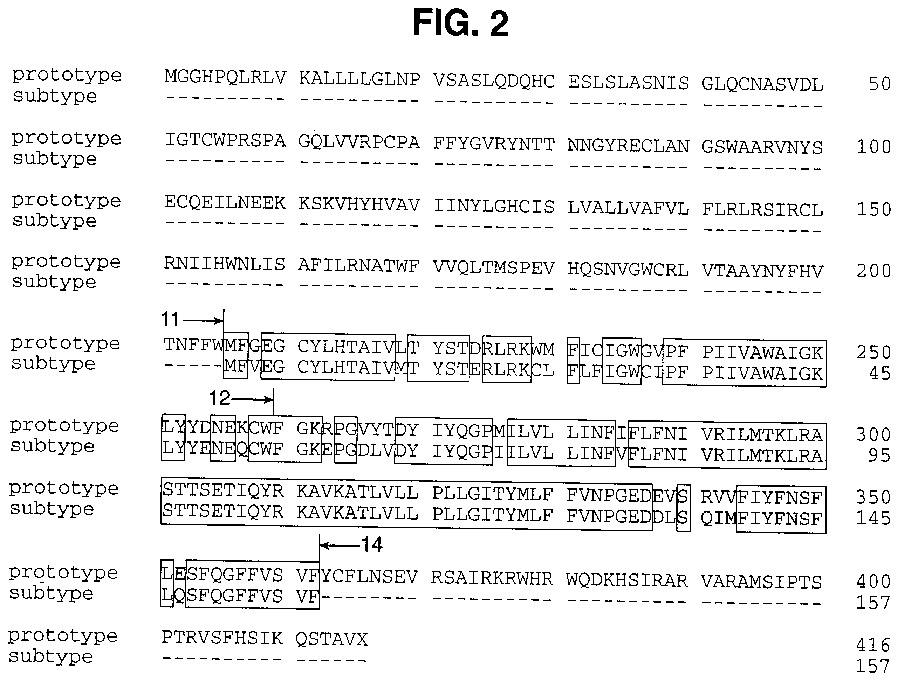

Fig. 2. Alignment of the amino acid sequence predicted by the open reading frame of the human CRH receptor related cDNA with that of the human CRH receptor clone. The single open reading frame in the CRH receptor related cDNA was translated, and the resulting translation (subtype) was aligned with the entire predicted amino acid sequence of the human CRH receptor (Chen et al., 1993) labeled "prototype". Identities between the two sequences are boxed and within the regions compared they show 85% homology. The arrows show the locations of the degenerate primers that were used to amplify cDNAs related to the CRH receptor.

The CRH receptor shows relatively little homology with other members of the G protein-coupled receptor superfamily (Chang et al., 1993; Chen et al., 1993; Vita et al., 1993). However, there are regions in which some amino acids are conserved between the CRH receptor and its closest relatives, the secretin, vasoactive intestinal peptide, and growth hormone releasing factor receptors. We designed degenerate primers that were based solely on the amino acid sequence of the human CRH receptor (Chen et al., 1993), and ended at amino acid residues that were absolutely conserved with its closest relatives. We also chose the locations of these primers based on a prevalence of amino acids that are encoded by few codons, which allowed the design of mixed oligonucleotides with a low degeneracy. The locations of the amino acid sequences used in designing the degenerate oligonucleotide primers are shown in Fig. 2.

Primers 11 and 14 were used to amplify cDNAs from human skeletal muscle and brain in polymerase chain reactions as described under Materials and Methods. The resulting material was reamplified using primers 12 and 14. After the two sequential PCR reactions, discrete cDNA bands could be visualized after electrophoresis on agarose gels. The amplified cDNA was cloned in the vector pCRII, and the nucleotide sequence of the inserts were determined. The majority of the clones proved to be identical to the published sequence of the human CRH receptor. Two clones derived from brain cDNA and one from skeletal muscle cDNA, however, while similar to the CRH receptor sequence, showed significant differences. Furthermore, the sequences of these new clones were essentially identical to each other. A CRH receptor-related receptor specific PCR primer, primer 28, complementary to bases 186-202 of the CRH receptor-related sequence in Fig. 1, was synthesized. Primer 28 was used in conjunction with primer 11 to reamplify the products of amplifying human brain cDNA with primers 11 and 14. The resulting amplified cDNA was cloned into the vector pCRII, and the sequences of the cDNA inserts in several clones was determined. The sequences of the 11/28 fragments overlapped the primer 12/14 fragments, allowing the assembly of the 471 bp sequence shown in Fig. 1. An alignment of the CRH receptor-related cDNA with the sequence of the human CRH receptor shows that the two sequences are 82% homologous. This cDNA sequence consists entirely of an open reading frame that predicts 157 amino acid residues that are highly homologous in sequence to the human CRH receptor amino acid sequence (Fig. 2).

Clones of the sequences of the 12/14 and 11/28 fragments were named phCRHRR-1 and phCRHRR-2, respectively, and were incorporated into DH5α cell lines. These cell lines, DH5α [phCRHRR-1] and DH5α [phCRHRR-2] are being deposited in the American Type Culture Collection (12301 Parklawn Drive, Rockville, Maryland 20852) as an example of this invention.

Although the current clones do not represent a full-length cDNA, they do provide sufficient information to allow the isolation of such a clone by standard methods such as the screening of cDNA libraries with radioactively labeled probes or through the use of PCR techniques such as RACE (Rapid Amplification of cDNA Ends, Frohman, et al., Proc. Nat'l. Acad. Sci. U.S.A., 85, 8998-9002, 1988). Kits for the RACE technique are commercially available from suppliers such as GIBCO BRL. We have also designed primers that allow the specific amplification of the human CRH receptor-related cDNA (Primers 32A and 32B, Materials and Methods) that can be used to detect transcription of this gene through the method of reverse transcription-PCR (RT-PCR). This entails isolation of RNA from tissues or cells, reverse transcription of the mRNA, followed by PCR amplification using the gene specific primers 32A and 32B. We have tested these primers on the cloned human CRH receptor and the human CRH receptor-related cDNA described here, and have shown that these oligonucleotides specifically amplify the human CRH receptor-related cDNA when used as PCR primers. This could be accomplished using as primers other portions of the CRH receptor-related sequence that differ from the prototype CRH receptor sequence.

A general utility of the novel human CRH receptor-related gene is to screen for therapeutic drugs with a greater specificity of action or fewer side effects than are attainable by screening with the prototype CRH receptor alone. These therapeutic compounds would have utility in the treatment of depression, anxiety, inflammation, inflammatory bowel disease, and HIV related dementia. The cloned CRH receptor-related cDNA makes it possible to isolate full length cDNA or genomic clones that can be used to express the encoded novel receptor in active form. The recombinantly expressed receptor can be used to screen compounds for their ability to bind to the receptor and to affect signal transduction by this novel receptor.

The CRH receptor-related sequence can be used to develop subtype specific transcriptional assays useful in determining the tissue specific expression pattern of the prototype and potential subtype CRH receptors. This can be through reverse transcription PCR (RT-PCR), in situ hybridization or in situ transcription approaches ("In Situ Hybridization in Neurology. Advances in Methodology", edited by Eberwine et al., 3-192, Oxford University Press, N.Y., 1994). This information is useful in determining the involvement of these receptors in various diseases and in developing novel and improved therapies.

The human CRH receptor related partial cDNA is sufficient to be useful in isolating genomic clones from human or other mammalian DNAs. Homologous CRH receptor related clones from other species would be useful in developing disease models, particularly the mouse gene, which could be used to interrupt the gene in transgenic mice. Expression of the endogenous CRH receptor related gene could also be regulated through he use of antisense oligonucleotides or by expression in cells or animals of the antisense strand of the cDNA by means of a recombinant vector.

Gene expression can be modulated through the use of antisense oligonucleotides or RNA. Antisense oligonucleotides (usually 18-25 bases in length) complementary to an RNA of interest are synthesized. Generally this sequence is located within the coding region of the mRNA. Treatment of cells in culture or in vivo results in a reduction of the amount of protein expressed by the targeted mRNA. This is particularly useful in discovering the function of receptors in the central nervous system, where loss of function can produce observable behavioral changes. The antisense oligonucleotides can be introduced into the cerebro-spinal fluid of an animal, where they specifically interfere with the synthesis of the protein encoded by the mRNA of interest in the brain or spinal chord. This method is described by Wahlestedt et al., Science, 2595, 526-31 (1993) and Standifer et al., Neuron, 12, 805-810 (1994). These references are incorporated herein by reference in their entirety.

Gene expression can be ablated in transgenic mice. This process is useful in discovering gene function and developing disease models useful in discovering and characterizing therapeutic compounds. This method consists of isolating a genomic clone for the gene of interest from mouse genomic DNA. The clone does not have to contain the entire gene. The gene is then interrupted by the insertion of a foreign DNA segment, usually a selectable antibiotic resistance gene. The interrupted gene is then introduced in embryonic stem cells where it recombines with the host cell DNA. Embryonic stem cells carrying the interrupted gene are introduced into mouse blastocysts, which are then transferred into a foster mother. Chimeric mice are identified among the young and are bred to create lines of mice carrying the interrupted gene. This approach is currently most practical in but not necessarily limited to mice. This approach is described by Rossant, J., Neuron, 2, 323-334 (1990) and Li et al., Cell, 76, 427-437 (1994). These references are incorporated herein by reference in their entireties.

The novel peptide sequence of this invention may also be used to produce monoclonal and polyclonal antibodies against a CRH receptor-related receptor that comprises such sequence or afunctionally equivalent peptide sequence. Oligopeptides are synthesized based upon the amino acid sequence predicted by the nucleotide sequence determined for a gene of interest. These oligopeptides are used to immunize animals, thus producing antibodies. Antibodies that discriminate between closely related proteins can be generated by choosing regions of amino acid sequence that differ between the two proteins. This method can be applied to the generation of monoclonal and polyclonal antibodies. These antibodies may be useful in diagnosis, localization of specific proteins, and protein structure-function studies.

Briefly, the amino acid sequence of a gene of interest is predicted based upon the nucleic acid sequence that has been determined from genomic or cDNA clones. A portion of the predicted amino acid sequence is chosen to be used as an antigen. Several criteria for this choice are possible. For example, the amino acid sequence may discriminate between the gene product of interest and other related proteins, the amino acid sequence may occur in a region of the particular interest, or the sequence may be exposed to the cell interior or exterior. The desired oligopeptide is chemically synthesized and then used to immunize animals to generate antibodies. The oligopeptide may be used as is or may be chemically crosslinked to a larger carrier protein such as keyhole limpet hemocyanin (KLH).

Antibodies raised in the animal are then characterized and optionally purified from serum using standard immunological techniques. In the case of monoclonal antibodies, spleen cells from an immunized animal, commonly a mouse, are fused to myeloma cells. The resultant hybridoma cells are then characterized for the secretion of a monoclonal antibody with the desired binding characteristics. An alternative method of generating antibodies that bind to the protein of interest is to fuse a portion of the DNA sequence encoding the protein of interest to a known, foreign protein-encoding gene segment. The resultant fusion gene, carried in a vector with the appropriate signals to allow expression of the fusion gene product, is introduced into the host cell.

The expressed fusion protein is purified, usually taking advantage of the properties of the known portion of the fusion protein. These properties may include binding to an affinity matrix or possessing antigenic determinants recognized by an existing antibody. The purified fusion protein is then used to generate antibodies as described above. These methods are described by Doolittle, R.F., "Of URFS and ORFS: A Primer On How To Analyze Derived Amino Acid Sequences", University Science Books, Mill Valley, CA (1986) and Marty et al., "Transmembrane Orientation of the N-terminal and C-Terminal Ends of the Ryanodine Receptor in the Sarcoplasmic Reticulum of Rabbit Skeletal Muscle, Biochem J., 298, 743-749 (1994). These references are incorporated herein by reference in their entireties.

### Materials and Methods

### Reverse Transcription

First strand cDNA was synthesized by reverse transcription of human brain and skeletal muscle poly(A)+ RNA (obtained from Clontech) using Superscript II MMLV reverse transcriptase (BRL) and oligo d(T)₁₂₋₁₈ as the primer according to the supplier's instructions.

### Oligonucleotide Primers

Oligonucleotide primers were ordered from Genosys Biotechnologies, Inc. Degenerate oligonucleotides were designed based upon the sequence of the previously described human CRH receptor (Chen, et al., 1993). Bases within brackets indicates the bases present at a given position in the mixture of oligonucleotides that compose the degenerate oligonucleotide. I = inosine, which was used instead of a mixture of all four bases at a single position.

Primer #11 5′-CA[C,T]GTIACIAA[C,T]TT[C,T]TT[C,T]TGG-3′

Primer #12 5′-TA[C,T]GA[C,T]AA[C,T]GA[A,G]AA[A,G]TG[C,T]TGG-3′

Primer #14 5′-AC[C,T]TCI[C,G][A,T][A,G]TTIA[A,G][A,G]AA[A,G]CA[A,G]TA-3′

Human CRH Receptor-related cDNA specific primers were generated based on the sequences of putative CRH receptor subtype partial cDNA clones. These were:

Primer #28 5′-GGTAGATGTAGTCCACCAGG-3′

Primer #32A 5′-GTTTGGCAAGGAGCCTGGCGAC-3′

Primer #32B 5′-CATGATCTGTGACAGGTCG-3′

### PCR and Analysis of PCR Products

200 µM dNTPs
10 mM Tris pH 8.3
50 mM KCl
2.5 mM MgCl₂
Taq DNA polymerase, 1-2 units per 50 µl reaction

Each primer was present at 0.5-1.0 µM.

The first round of PCR was performed using the cDNA produced by reverse transcription of 600 ng of poly(A)+RNA and primers #11 and #14 with the following cycle conditions:
94°C, 3 min. - 1 cycle
(94°C, 30 sec.; 45°C, 1 min.; 72°C, 45 sec.) - repeated for 35 cycles
72°C, 5 min. - 1 cycle

The second round of PCR was performed using 2 µl of the product of the first round PCR as template and primers #12 and #14 or primers #11 and #28. The PCR cycle conditions were as in the first round.

PCR products were cloned using invitrogen's TA Cloning Kit according to the manufacturers instructions. The plasmid vector supplied in this kit is PCRII. The products of ligating the PCR products into PCRII were transformed into the E. coli host DH5α (GIBCO BRL), and plasmid containing clones were selected by growth on plates containing PenG at 300 µg/ml.

Plasmid clones were submitted to the sequence analysis facility for nucleotide sequence determination on ABI model 373A fluorescent automated sequencers. The sequence data was analyzed by comparison to the nucleotide sequence data base using the BLAST program. (See Altschul et al., "Basic Local Alignment Search Tool", J. Mol. Biol., 215, 403-410 (1990)).

## Claims

1. An isolated DNA segment comprising the DNA sequence of SEQUENCE ID NO. 1, or an allelic variation of such sequence.

2. An isolated DNA segment that is a subset of, and functionally equivalent to a DNA segment according to claim 1.

3. A polypeptide produced by expression in a host cell into which has been incorporated the DNA sequence of SEQUENCE ID NO. 1 or an allelic variation of such sequence.

4. A substantially purified polypeptide comprising the amino acid sequence of SEQUENCE ID NO. 2.

5. A substantially purified polypeptide that is a subset of, and functionally equivalent to a polypeptide comprising the amino acid sequence of SEQUENCE ID NO. 2.

6. Recombinant DNA comprising a DNA segment according to claim 1.

7. A DNA vector comprising recombinant DNA according to claim 6.

8. A host cell into which has been incorporated recombinant DNA according to claim 6.

9. A DNA segment comprising a DNA sequence that is a subset of the DNA sequence of SEQUENCE ID NO. 1, or an allelic variation of such sequence, and that is capable of hybridizing to the DNA sequence of SEQUENCE ID NO. 1, or an allelic variation of such sequence, when used as a probe, or of amplifying all or part of such sequence when used as a polymerase chain reaction primer.

10. A DNA segment according to claim that comprises the DNA sequence of SEQUENCE ID NO. 3 or SEQUENCE ID NO. 4, or an allelic variation of one of these sequences.

11. A method of detecting the presence in human cells of mRNA that encodes a human CRH receptor-related receptor that comprises the peptide sequence of SEQUENCE ID NO. 2, or a functionally equivalent peptide sequence, said method comprising: (a) performing a reverse transcriptase-polymerase chain reaction on total RNA from human cells using a pair of polymerase chain reaction primers that are specific for such receptor, as determined from the DNA sequence of SEQUENCE ID NO. 1 or an allelic variation of such sequence, and (b) assaying the presence of an appropriately sized PCR fragment or hybridizing the products of such reaction to the DNA sequence of SEQUENCE ID NO. 1.

12. A method of identifying ligands that bind to or displace the binding of a known ligand to a human CRH receptor-related receptor that comprises the peptide sequence of SEQUENCE ID NO. 2, said method comprising determining the degree of binding of a substance to such receptor or determining the ability of a substance to displace the binding of a known ligand to such receptor in (a) a cell line into which has been incorporated recombinant DNA according to claim 6, or (b) a cell population or cell line that naturally selectively expresses such receptor.

13. A method of isolating a full length human CRH receptor-related receptor cDNA or a genomic human CRH receptor-related receptor clone comprising using the DNA sequence of SEQUENCE ID NO. 1 or an allelic variation thereof, or a subset of such sequence or allelic variation, as a probe to screen a human cDNA or genomic library, or as a polymerase chain reaction primer.

14. A method of modulating expression in a human cell of a human CRH receptor-related receptor that comprises the DNA sequence of SEQUENCE ID NO. 1, said method comprising synthesizing one or more antisense oligonucleotides complimentary to the coding region of human CRH receptor-related receptor mRMA, and incorporating said antisense oligonucleotides into a human cell line in culture or in vivo.

15. A method of producing a monoclonal or polyclonal antibody against a CRH receptor-related receptor that comprises the peptide sequene of SEQUENCE ID NO. 2, comprising: (a) synthesizing an oligopeptide that is a subset of SEQUENCE ID NO. 2 or a functionally equivalent peptide, optionally crosslinking such oligopeptide to a larger carrier polypeptide, and immunizing an animal with said oligopeptide or with such crosslinked polypeptide; or (b) fusing an oligonucleotide that corresponds to and expresses an oligopeptide that comprises the peptide sequence of SEQUENCE ID NO. 2 or a functionally equivalent peptide to a larger DNA sequence that expresses a polypeptide to form a fused DNA sequence, expressing the fused protein encoded by said fused DNA sequence in an appropriate host cell, and immunizing an animal with said fused protein.

16. A process comprising inserting into an embryonic stem cell of a mouse or other mammal a genomic clone for a gene comprising a DNA segment according to claim 1, in which said gene has been interrupted by the insertion of a foreign DNA segment.

17. A process comprising introducing an embryonic stem cell carrying a gene comprising a DNA segment according to claim 1 that has been interrupted by the insertion of a foreign DNA segment into a mouse or other mammal blastocyst, and then introducing said blastocyst into a foster mother.
